(19) Europäisches Patentamt
European Patent Office
Office européen des brevets

(11) **EP 2 006 296 A1**

(12) **EUROPEAN PATENT APPLICATION**
published in accordance with Art. 153(4) EPC

(43) Date of publication:
**24.12.2008 Bulletin 2008/52**

(21) Application number: **07738461.8**

(22) Date of filing: **13.03.2007**

(51) Int Cl.:
*C07K 14/435* (2006.01)     *A23J 1/04* (2006.01)
*A23J 3/04* (2006.01)     *A23L 3/36* (2006.01)
*A23B 4/08* (2006.01)     *A23L 1/10* (2006.01)
*A23L 1/16* (2006.01)     *A23L 1/32* (2006.01)
*A23L 1/325* (2006.01)

(86) International application number:
**PCT/JP2007/054989**

(87) International publication number:
**WO 2007/105734 (20.09.2007 Gazette 2007/38)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.03.2006 JP 2006067758**

(71) Applicant: **NIPPON SUISAN KAISHA, LTD.
Tokyo 100-8686 (JP)**

(72) Inventors:
• **CHIBA, Satoru
Hachioji-shi, Tokyo 192-0906 (JP)**
• **KUBOTA, Mitsutoshi
Hachioji-shi, Tokyo 192-0906 (JP)**
• **NISHIZAWA, Satoko
Hachioji-shi, Tokyo 192-0906 (JP)**
• **OKAMOTO, Naoko
Hachioji-shi, Tokyo 192-0906 (JP)**
• **SUZUKI, Kenichi
Hachioji-shi, Tokyo 192-0906 (JP)**

(74) Representative: **Vossius & Partner
Siebertstrasse 4
81675 München (DE)**

(54) **CRUSTACEAN-DERIVED PROTEIN HAVING ANTIFREEZE ACTIVITY**

(57)     A crustacean-derived protein having antifreeze activity which under reduced conditions has a molecular weight, as measured by sodium dodecyl sulfate (SDS) - polyacrylamide gel electrophoresis, of about 37,000, about 16,000, and/or about 15,400; and the N-terminal amino acids are indicated by SEQ ID No. 1 or SEQ ID No. 2.

Since this protein is excellent in comparison to the conventional antifreeze proteins from the standpoints of safety, activity, production rate, cost, and the like, use is possible in a wide range of fields, beginning with foods.

**EP 2 006 296 A1**

**Description**

[TECHNICAL FIELD]

The present invention relates to a protein that has antifreeze activity, a production method for the protein, and a use of the protein.

[BACKGROUND ART]

Although water is said to become ice at 0°C, more specifically, the phenomenon of supercooling is known to occur when pure water is cooled to 0°C without the beginning of freezing, and where freezing does not occur even at temperatures below the freezing point. When the temperature becomes less than or equal to 0°C, some substances present in the water become nuclei for ice, and freezing of water proceeds by the process of crystal growth. If this freezing phenomenon can be controlled, applications are thought to be possible in fields beginning with foodstuff freezing preservation technology, microorganism and biological tissue storage technology, and the like as well as various fields such as artificial rainfall stimulation technology, cryogenic shipping technology, and the like.
Attention has been paid to proteins as substances related to freezing of water, and such proteins are ice nucleation proteins (INP) and antifreeze proteins (AFP).
Ice nucleation activity bacteria are bacteria that have the ability to cause pure water, which does not freeze even at -20°C, to freeze at -2°C to -4°C, and thus ice nucleation activity bacteria clearly act as nuclei during the start of freezing of water. Such bacteria are known to cause frost damage in plants. Bacteria known to be such ice nucleating bacteria belong to the genus Pseudomonas and the genus Erwinia (Non-Patent Documents 1 and 2). An ice nucleation protein is a protein having ice nucleation activity that is obtained from these ice nucleating bacteria. The majority of ice nucleation proteins are derived from microorganisms and, although their utilization has been proposed in the field of foods (Patent Documents 1 - 4), these ice nucleation proteins have actually not been put into much practical use.
An antifreeze protein is a protein that displays activity that blocks the growth of ice crystals, and these proteins are produced by most species of living things living in low temperature environments. Such species of living things include fish living in the Antarctic Ocean or in low temperature environments, plants living in cold regions, larvae of beetles that overwinter in cold regions, microorganisms that adapt to low temperature, and the like. Plant-derived antifreeze proteins (Patent Documents 1 and 2), lichen-derived antifreeze proteins (Patent Document 2), fish-derived antifreeze proteins (Patent Documents 3 and 4), insect-derived antifreeze proteins (Patent Document 5), and the like have been previously reported. Even though antifreeze proteins have been obtained from such a wide range of species of living things, crustacean-derived antifreeze proteins have not been reported.

[Patent Document 1] Published International Application WO98/04148
[Patent Document 2] Published International Application WO99/37673
[Patent Document 3] Published International Application WO96/11586
[Patent Document 4] Published International Application WO97/02343
[Patent Document 5] Published International Application WO99/00493
[Patent Document 6] Published International Application WO90/13571
[Non-Patent Document 1] Appl. Microbiol., 28, p. 456, (1974)
[Non-Patent Document 2] Proc. 4th Int. Cont. Plant. Path. Bact., p. 725, (1978)

[DISCLOSURE OF THE INVENTION]

[PROBLEM TO BE SOLVED BY THE INVENTION]

The problem for the present invention is to provide a substance having antifreeze activity that is capable of use in a wide range of fields, beginning with foods, from the standpoints of safety, activity, production rate, cost, and the like.

[MEANS FOR SOLVING THE PROBLEM]

Although experiments were started based on the thought of the inventors of the present invention that antifreeze protein might be contained in krill which inhabit the Antarctic ocean since such antifreeze proteins have been known from many types of organisms, a substance having antifreeze activity could not be obtained by methods such as those mentioned in commonly known references. By devising an extraction method, a protein was discovered that had antifreeze activity and a protein was also discovered that had ice nucleation activity. Furthermore, as a result of paying attention also to non-krill crustaceans, the inventors of the present invention succeeded in obtaining similar substances by adoption of the same extraction method.

The present invention provides proteins (1) through (3) having antifreeze ability:

(1) a crustacean-derived protein having antifreeze activity;
(2) the protein having antifreeze activity according to (1), wherein the crustacean-derived protein, as measured by molecular weight measurement using sodium dodecyl sulfate (SDS) - polyacrylamide gel electrophoresis, has reduced-form molecular weights of about 37,000, about 16,000, and/or about 15,400 and the N-terminal amino acids are indicated by SEQ ID No. 1 or SEQ ID No. 2; and
(3) the protein having antifreeze activity according to (1) or (2), wherein the crustacean is a shrimp, crayfish, crab, krill, or mantis shrimp.

The present invention provides a crustacean extract having antifreeze activity according to (4) - (6):

(4) a crustacean extract having antifreeze activity and containing a crustacean-derived protein having antifreeze activity;
(5) the crustacean extract having antifreeze activity of (4), wherein the crustacean-derived protein, as measured by molecular weight measurement using sodium dodecyl sulfate (SDS) - polyacrylamide gel electrophoresis, has reduced-form molecular weights of about 37,000, about 16,000, and/or about 15,400 and the N-terminal amino acids are indicated by SEQ ID No. 1 or SEQ ID No. 2; and
(6) the crustacean extract having antifreeze activity of (4) or (5), wherein the crustacean is a shrimp, crayfish, crab, krill, or mantis shrimp.

The present invention provides a production method for a crustacean extract having antifreeze activity according to (7) - (13):

(7) a production method for a crustacean extract having antifreeze activity characterized by performing hot water extraction from the shells, meat, and/or internal organs of the crustacean;
(8) the production method for a crustacean extract according to (7), wherein the hot water extraction is performed using hot water of a temperature greater than or equal to 60°C;
(9) a production method for a crustacean extract having antifreeze activity, wherein the production method characterized by performing heat treatment and/or reduction treatment of a body liquid obtained by compressing the meat and/or internal organs of the crustacean or of an extract liquid of the shells, meat, and/or internal organs.
(10) the production method for a crustacean extract having antifreeze activity according to (9), wherein the body liquid obtained by compression of the meat and/or internal organs of the crustacean or the extract liquid of the shells, meat, and/or internal organs, as measured by molecular weight measurement using sodium dodecyl sulfate (SDS) - polyacrylamide gel electrophoresis, has a non-reduced-form molecular weight of about 200,000 and displays reduced-form bands at about 86,000 and 90,000, wherein the N-terminal amino acids are indicated by SEQ ID No. 3 or SEQ ID No. 4;
(11) the production method for a crustacean extract having antifreeze activity according to (9) or (10), wherein extraction from the shells, meat, and/or internal organs of the crustacean is performed by extraction using an extraction liquid having an added surfactant;
(12) the production method for a crustacean extract having antifreeze activity according to (9), (10), or (11), wherein the heat treatment is performed at a temperature greater than or equal to 60°C and the reduction treatment is performed by addition of a reducing agent; and
(13) the production method for a crustacean extract having antifreeze activity according to any one of (7) through (12), wherein the crustacean is a shrimp, crayfish, crab, krill, or mantis shrimp.

The present invention provides a food quality improvement agent that contains the protein having antifreeze activity or the crustacean extract having antifreeze activity and provides a food to which this quality improvement agent has been added, (14) through (17):

(14) a food quality improvement agent that includes: the protein having antifreeze activity of any one of (1) through (3), the crustacean extract having antifreeze activity of any one of (4) through (6), or the crustacean extract having antifreeze activity produced by the method of any one of (7) through (13);
(15) a food of improved quality to which has been added the quality improvement agent of (14);
(16) the food according to (15), wherein the food is a frozen food; and
(17) the food according to (15) or (16), wherein the food uses as a egg, wheat flour, or fish meat raw material.

[ADVANTAGES OF THE INVENTION]

The protein having antifreeze activity of the present invention is a protein derived from crustaceans that are suitable for addition to food. Thus, addition is possible to various types of food. In particular, by addition to frozen food, deterioration of quality due to freezing of the frozen food can be suppressed. Moreover, since the protein is contained in large amounts even in shells which are discarded, the present invention makes use of a waste product and the protein can be produced inexpensively.

[BRIEF DESCRIPTION OF THE DRAWINGS]

FIG. 1 shows a chromatography chart of the protein extracted from krill of Working Example 1.
FIG. 2 shows a SEPHACRYL S-200 HR gel filtration chromatography chart of Working Example 1.
FIG. 3 shows a SEPHACRYL S-200 HR gel filtration chromatography chart of Working Example 1.
FIG. 4 is a photograph of electrophoresis using a SDS-polyacrylamide gel to check molecular weight of the present protein sample 1.
FIG. 5 is a photograph of electrophoresis using a SDS-polyacrylamide gel to check molecular weight of the present protein sample 2.
FIG. 6 shows measured results of antifreeze activity of Working Example 4.
FIG. 7 is a photograph showing bi-pyramidal shaped ice crystals during testing to measure antifreeze activity of Working Example 4.
FIG. 8 shows temperature stability of antifreeze activity of the present protein sample 2.
FIG. 9 shows results of measurement of recrystallization inhibition activity of the present protein sample 2.
FIG. 10 is a photograph of results of SDS-polyacrylamide gel electrophoresis using analysis of extract powders A and B.
FIG. 11 is a chart showing results of measurement of antifreeze activity of extract powders A and B.
FIG. 12 is a photograph of a hexagonal-planar shaped ice crystal during testing to measure antifreeze activity of extract powder A.
FIG. 13 is a photograph of a hexagonal-planar shaped ice crystal during testing to measure antifreeze activity of extract powder B.
FIG. 14 is a chart showing results of measurement of recrystallization inhibition activity for ice crystals of the extract powders A and B.
FIG. 15 shows the SUPERDEX G30 gel filtration chromatograph chart of protein extracted from dry krill powder of Working Example 8.
FIG. 16 shows the reversed phase HPLC chart of Working Example 8.
FIG. 17 shows the reversed phase HPLC chart of Working Example 8.
FIG. 18 shows the syneresis inhibition effect of the krill-derived extract powder in starch gel (within the figure, "shells" indicates data using the krill shell-derived extract powder B, and "whole" indicates data using the krill-derived extract powder A).
FIG. 19 is a photograph showing the starch gel (stored frozen for 7 days) free of added extract powder as observed using an electron microscope.
FIG. 20 is a photograph showing the starch gel (stored frozen for 7 days) to which the extract powder A was added as observed using an electron microscope.
FIG. 21 shows salt-induced solubility of minced meat to which the extract powder B had been added.
FIG. 22 shows the amount of generated DMA of minced meat to which the extract powder B had been added.

[BEST MODE FOR CARRYING OUT THE INVENTION]

In the present invention, the term "crustacean" indicates the Crustacea class of the Pancrustacea subphylum of the Arthropoda phylum, and this term further indicates an animal classified as belonging to the Cepharocarida subclass, Remipedia subclass, Branchiopoda subclass, Maxillopoda subclass, or Malacostraca subclass. Examples that can be cited are: shrimps, crayfishes, and crabs included in the order Decapoda; northern krill and Antarctic krill of the order Euphausiacea; mantis shrimps of the order Stomatopoda; and the like, which are frequently used as food in the seafood industry.
In the present invention, antifreeze activity is evaluated by thermal hysteresis. Although the freezing temperature of water and the solidification point are normally identical, in the presence of a substance such as an antifreeze protein, the protein binds to the surface of ice crystals, blocks growth of the crystals, lowers the solidification point, and then results in a difference (i.e. thermal hysteresis) between the freezing temperature and the melting temperature. In the working examples of the present specification document, antifreeze ability has been evaluated by thermal hysteresis by finding the solidification point of a solution using an osmometer (e.g., osmometer OM 801, manufactured by Vogel Corp.).

When frozen water is slightly melted by a rise of temperature, phenomena occur such as the frozen water freezing again around residual ice crystals such that the ice crystals gradually grow larger and the small ice crystals bond together to form large ice crystals. These phenomena are called recrystallization of ice crystals. The expression "recrystallization inhibition activity for ice crystals" is taken to mean activity that suppresses this recrystallization. That is to say, this activity maintains unchanged the small size of small crystals. The antifreeze protein of the present invention has this recrystallization inhibition activity for ice crystals, and this antifreeze protein of the present invention is capable of suppressing deterioration of quality of foods due to temperature changes during frozen storage, not just during freezing of the frozen food and the like.

In the present invention, the presence or absence of recrystallization inhibition activity for ice crystals was determined by observation of ice crystals using a microscope and observing the count of fine ice crystals. That is to say, 2 $\mu$L of a protein sample having recrystallization inhibition activity for ice crystals dissolved in a 30% sucrose solution is placed drop-wise on a cover glass, and another cover glass is placed thereon to sandwich the solution therebetween. The cover glass assembly is placed on the stage of an optical microscope (model BH2 microscope, produced by Olympus Corp.; and LK600 temperature controller, produced by Linkam Scientific Instruments Ltd.) that is capable of temperature control, and heating and cooling are performed repeatedly at a rate of 0.1°C/second, in order, to temperatures of 20°C, -30°C, 20°C, -30°C, 20°C, -30°C, and -10°C. Then temperature is held at -10°C for 30 minutes, and the count is determined of fine ice crystals having a surface area of 10.01 to 35 $\mu$m$^2$. Although ice crystals grow large and the number of fine ice crystals becomes small when there is no addition of a substance that has recrystallization inhibition activity for ice crystals, the number of fine ice crystals becomes high when there is recrystallization inhibition activity for ice crystals.

In order to produce protein having antifreeze activity of the present invention, the protein is firstly extracted from crustaceans. General methods can be used for extraction, separation, and concentration of the protein. That is to say, all the tissue present in the crustacean is crushed, the obtained suspension liquid is subjected to centrifugal separation, and insoluble substances are removed. At this time, solubilization is accelerated and extraction ability is increased by use of a surfactant (cationic type, non-ionic type, amphoteric type, anionic type, high molecular weight surfactant and the like). A non-ionic surfactant is particularly suitable. Protein can be separated and concentrated from the obtained supernatant by the following indicated general methods: separation and concentration methods using ammonium sulfate fractionation as a salting-out method; methods using acetone, ethanol, propanol, or methanol for separation and concentration by an organic solvent; acidic precipitation methods using hydrochloric acid, sulfuric acid, or trichloroacetic acid; separation and concentration methods utilizing a water soluble polymer (polyethylene glycol or dextran); separation and concentration methods using ultrafiltration (membrane concentration); and adsorption and separation on an ion exchange chromatography, hydrophobic interaction chromatography, hydroxyapatite chromatography, reversed phase chromatography, or gel filtration chromatography. The supernatant can also be subjected to heat treatment to cause denaturation and precipitation of other proteins. The obtained isolate can be made into a powder by freeze drying, spray drying, and the like. Furthermore, the protein having antifreeze activity can be separated out using the property of absorption on the surface of ice crystals by using ice crystals to separate the protein from an aqueous solution containing the protein.

The protein displaying antifreeze ability is generated by subjecting the protein obtained by the above mentioned methods to heat treatment, reduction treatment, or thermal reduction treatment. Antifreeze ability is displayed by adding a reducing agent (e.g. 2-mercaptoethanol, dithiothreitol, ascorbic acid, and the like) and then heating. In order to obtain the protein having antifreeze ability, although the performance of heat treatment in addition to addition of a reducing agent is preferred at an appropriate time during extraction treatment or separation-concentration treatment, the protein having antifreeze ability can be obtained simply by performance of heat treatment without addition of the reducing agent.

A specific example of production includes crushing meat, internal organs, and/or shells of crustaceans (frozen, raw, or dry) to produce a suspension liquid. If this suspension liquid is then extracted using hot water, the antifreeze protein of the present invention can be obtained directly. Moreover, the antifreeze protein of the present invention can also be obtained by heat treatment or reduction treatment to cause decomposition of protein extracted at low temperature. At this time, the extraction temperature is preferably greater than or equal to 60°C and particularly preferably is greater than or equal to 80°C. Moreover, if extraction is performed at a low temperature of 0°C to 60°C, by subjecting the extraction liquid to heat treatment (greater than or equal to 60°C) or by treatment using a reducing agent (e.g. 2-mercaptoethanol, dithiothreitol, ascorbic acid, and the like), the antifreeze protein can be obtained by decomposition of the extracted protein.

The protein having antifreeze activity of the present invention includes proteins, as measured by molecular weight measurement using sodium dodecyl sulfate (SDS) - polyacrylamide gel electrophoresis, that display a reduced-form molecular weight of about 37,000, about 16,000, and/or about 15,400 and that display the N-terminal amino acids as indicated by SEQ ID No. 1 or SEQ ID No. 2. Although the above mentioned extraction liquid has antifreeze activity without further processing, if further purification and concentration is performed, such purification and concentration can be performed using these characteristic proteins as indicators.

Since the protein of the present invention has antifreeze ability and has recrystallization blocking ability for ice crystals, deterioration of food due to freezing can be suppressed by addition of the protein of the present invention to foods. Although the concentration during addition to food depends on the type of food and the object of use, the concentration added to the total food (at a degree of purity such as that of the present invention protein sample 2 of Working Example 1 and the various extract powders of Working Example 8) is about 0.00001% to 10% and preferably is about 0.0001% to 0.1%.

Although working examples of the present invention are described below, the present invention is not limited by these working examples.

[Working Example 1]

<Extraction and Purification of Protein Having Antifreeze Activity from Antarctic Krill (Whole)>

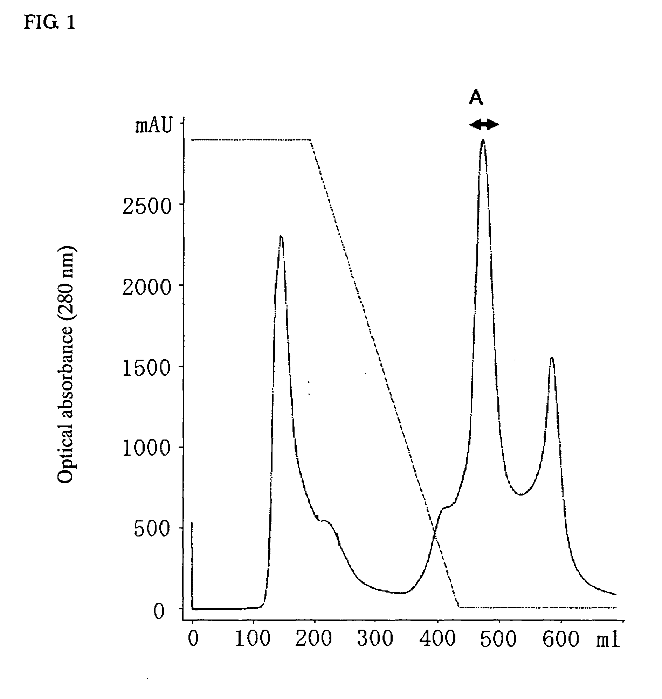

Using a homogenizer, 80 g of frozen Antarctic krill was suspended in 420 mL of an extraction liquid containing 50 mM ammonium hydrogen carbonate (pH 7.9) and 1 mM PMSF. Triton-X100 (produced by Sigma Corp.) was added to this suspension liquid to give a concentration of 0.1%, and the mixture was stirred on ice for 1 h. Thereafter, this suspension liquid was subjected to centrifugal separation for 30 minutes at 10,000 G, and the obtained supernatant was subjected to ammonium sulfate fractionation. That is to say, ammonium sulfate at 35 to 65% of saturation was added, and the obtained precipitate was centrifuged for 30 minutes at 10,000 G for separation and recovery. A solution containing 1 M ammonium sulfate and 50 mM ammonium hydrogen carbonate (pH 7.9) (solution A) was added to this precipitate to form a suspension liquid. The supernatant obtained by 20 minutes of centrifuging this suspension liquid at 10,000 G was applied for chromatography using a hydrophobic interaction column (TOYOPEARL Phenyl 650M, Tosoh Bioscience, Inc.; 2.6 cm $\times$ 20 cm (106 mL)) equilibrated with the solution A buffer solution, and protein was eluted by a linear gradient using 50 mM ammonium hydrogen carbonate (pH 7.9) (solution B). As a result, the A peak chromatography fraction shown in FIG. 1 was obtained.

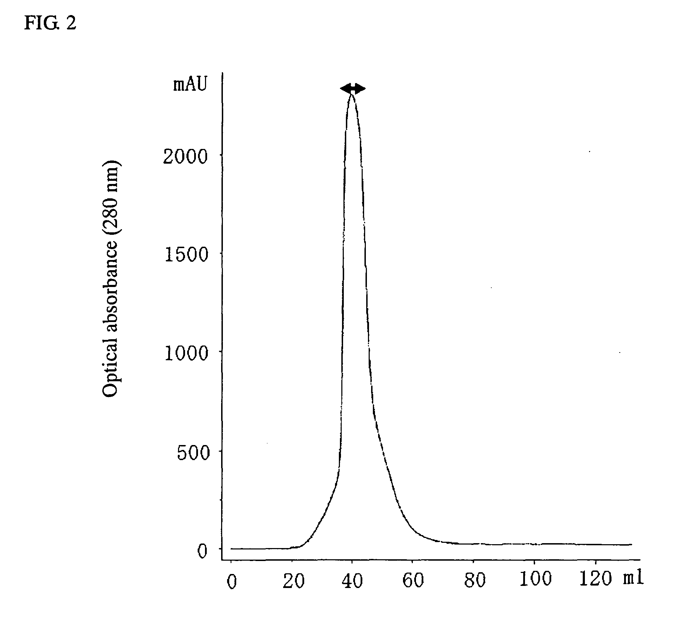

This fraction was placed in a dialysis membrane, the periphery was coated with polyethylene glycol 6000 (produced by Wako Pure Chemical Industries, Ltd.), the fraction was left for about 3 h at 4°C, and the fraction was concentrated roughly 10 fold. Then the resultant fraction was dialyzed against 10 mM ammonium hydrogen carbonate (pH 7.9) overnight at 4°C. After dialysis, the sample was applied for 1.6 cm $\times$ 60 cm SEPHACRYL S-200 HR gel filtration column chromatography (equilibrated beforehand with the same solution). The fraction of the peak indicated by the arrow in the chromatograph shown in FIG. 2 was obtained. This fraction was dialyzed against distilled water. Thereafter, the fraction was subjected to freeze drying. By use of this purification method, 80 mg of protein (present protein sample 1) was obtained from 80 g of frozen Antarctic krill.

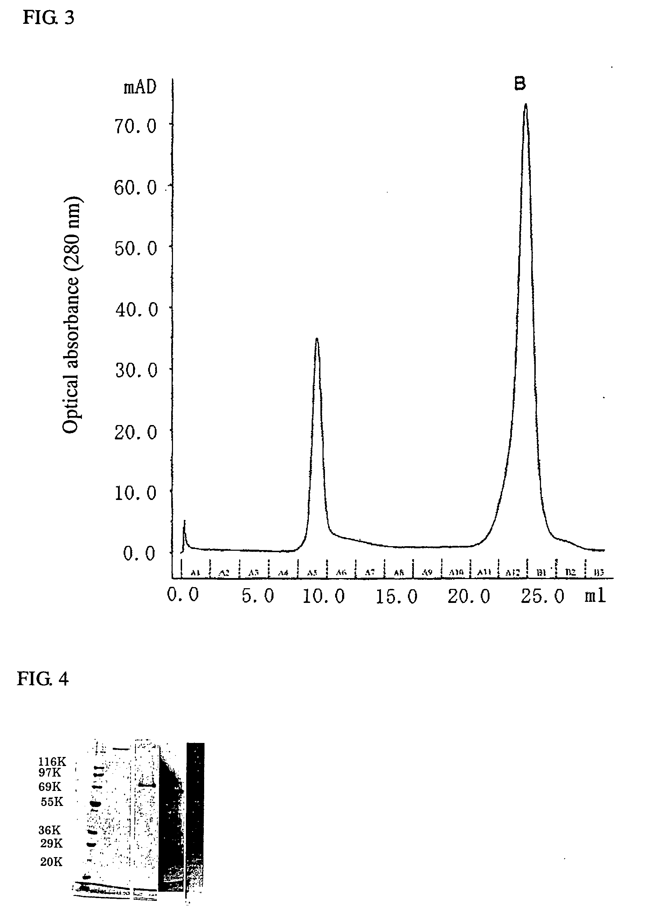

2-mercaptoethanol was added to the present protein sample 1 to give a final concentration of 1 mM, and the mixture was heated for 5 minutes at 85°C. This mixture was applied again for SEPHACRYL S-200 HR gel filtration column chromatography. As a result, peak B on the low molecular weight side appeared as shown in FIG. 3. This peak B fraction was collected and was concentrated by freeze drying. By this method, 70 mg of protein (present protein sample 2) was obtained from 102 mg of the present protein sample 1.

[Working Example 2]

<Check of Molecular Weight of Protein>

Molecular weight of the present protein sample 1 was measured by SDS-polyacrylamide gel electrophoresis. A 10% acrylamide gel and a buffer solution (pH 8.6) containing 0.1% SDS, 25 mM tris-hydroxymethyl aminomethane, and 192 mM glycine were used for about 2 h of electrophoresis at 12 mA. Thereafter, proteins were stained using CBB R-250. As a result, this protein was shown to have a subunit structure of about 200 kDa comprising monomers of about 86 kDa or 90 kDa (leftmost column of FIG. 4 shows molecular weight markers; the second column from the left shows the 200 kDa subunit; and the third column from the left shows the monomers at 86 kDa or 90 kDa). After the SDS-polyacrylamide gel electrophoresis, the presence of sugar chains was checked by immersion of the gel for 1 h at room temperature in 7.5% acetic acid solution. Thereafter, the gel was transferred to 0.2% periodic acid, and the gel was incubated for 45 minutes at 4°C. Then the gel was transferred to Schiff reagent (Wako Pure Chemical Industries, Ltd.), and the gel was refrigerated for 45 minutes. Thereafter, the Schiff reagent was removed, and the gel was washed using 10% acetic acid. As a result, the bands at 86 kDa, 90 kDa, and 200 kDa developed coloration showing that sugars were attached to these proteins (second column from the right in FIG. 4). Nile Blue A was used to check for fats. 0.25 g of Nile Blue A was dissolved in 100 mL of distilled water. Then 1 mL of concentrated sulfuric acid was added, and the mixture was boiled for 2 h. The mixture was filtered through a filter to produce the Nile Blue A solution. The post-electrophoresis gel was transferred to this solution, and the gel was incubated for 30 minutes at 50°C. Thereafter, the gel was transferred to a 5% acetic acid solution, and the gel was incubated for 2 days at 50°C. Then the gel was further incubated for 5 minutes in 0.5% hydrochloric acid solution, and the gel was then washed using distilled water. As a result, bands at 86 kDa, 90 kDa, and 200 kDa developed coloration showing that fatty acids were bonded to these proteins (rightmost column of FIG. 4).

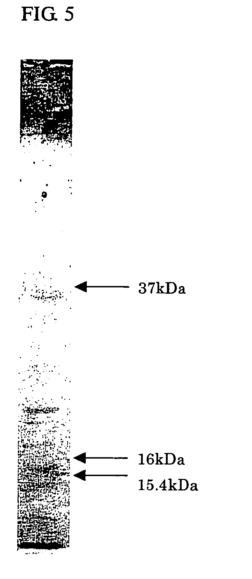

The present protein sample 2 was confirmed to have bands at 37 kDa, 16 kDa, and 15.4 kDa by 15 - 25% acrylamide gel electrophoresis (FIG. 5).

[Working Example 3]

The N-terminal amino acid sequence of the present protein sample 1 was analyzed. That is to say, after SDS-polyacrylamide gel electrophoresis was performed in the same manner as Working Example 2, a semi-dry type transfer apparatus was used for transfer to a polyvinylidene fluoride membrane. The bands of the present protein sample 1 were cut out, and the N-terminal amino acid sequences were analyzed using a protein sequencer (model 473A protein sequencer, produced by Applied Biosystems). As a result, the N-terminal amino acid sequences of the present protein sample were found to be those indicated by Protein SEQ ID Nos. 3 and 4 of the Sequence Listing. From this fact, isoforms having similar amino acid sequences are shown to be present in the present protein sample 1.

The N-terminal amino acid sequences were analyzed for the protein at 37 kDa of the present protein sample 2 in the same way. As a result, the N-terminal amino acid sequences of the present protein sample 2 were found to be those indicated by SEQ ID Nos. 1 and 2 of the Sequence Listing. From this fact, isoforms having similar amino acid sequences are shown to be present in the present protein sample 2.

[Working Example 4]

<Measurement of Antifreeze Ability>

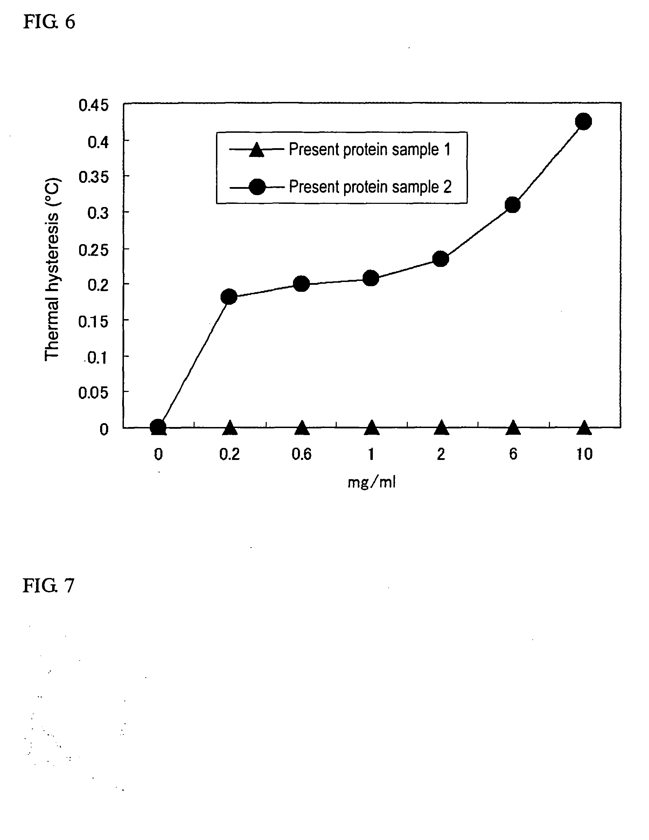

Antifreeze activity is evaluated by thermal hysteresis for the above mentioned present protein sample 1 and present protein sample 2. The solidification point of a solution were found using an osmometer OM 801 manufactured by Vogel Corp, then thermal hysteresis were found. As a result, the present protein sample 2 was found to raise thermal hysteresis in a concentration dependent manner over a protein concentration range of 0 to 10 mg/mL and was found to display thermal hysteresis of 0.42°C at a concentration of 10 mg/mL (FIG.6). On the other hand, increase of thermal hysteresis was not found for the present protein sample 1. This result shows that the low molecular weight protein generated by reduction has antifreeze activity.

Moreover, ice crystals were observed directly by microscope, and the presence or absence of antifreeze activity was determined based on whether or not there were characteristic changes of morphology (bi-pyramidal, hexagonal, and the like). 1 µL of the purified protein solution 1 was placed drop-wise on a cover glass, and another cover glass was placed thereon to sandwich the solution therebetween. The cover glass assembly was placed on the stage of an optical microscope (model BH2 microscope, produced by Olympus Corp.), an LK600 temperature controller produced by Linkam Scientific Instruments Ltd. was used to cool the interior of the stage at a rate of 0.1°C/second, and ice crystals were observed. As a result, ice crystals having a bi-pyramidal shape as shown in FIG. 7 were observed when the present protein sample 2 was added.

<Temperature Stability>

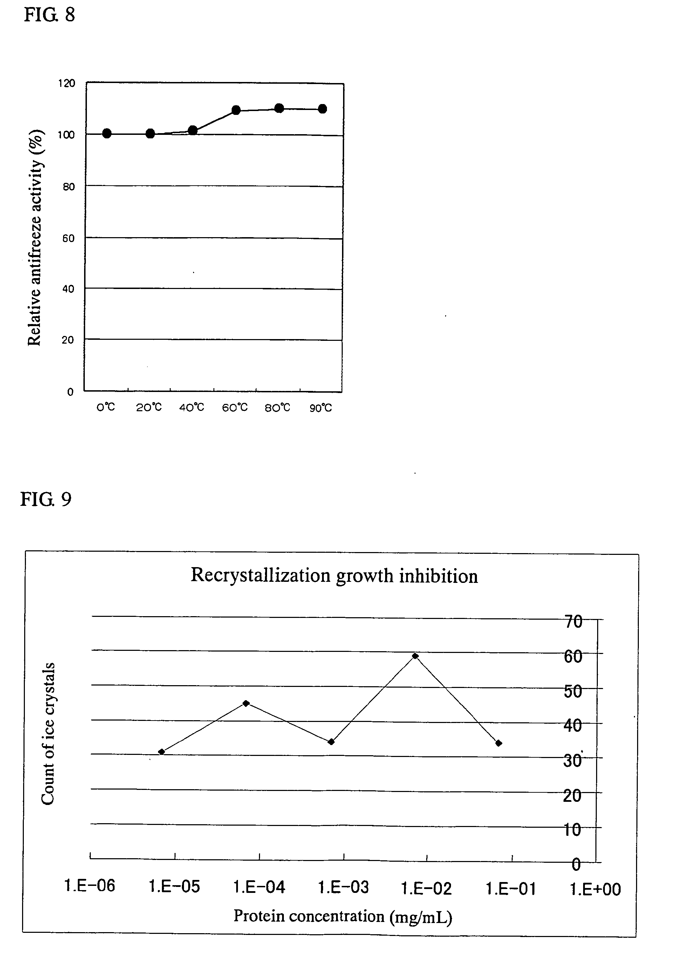

Temperature stability of the present protein sample 2 was tested. Various samples were dissolved in 50 mM ammonium hydrogen carbonate aqueous solutions (pH 7.9, 7.5 mg/mL protein concentration), and these solutions were held for 1 h at respective temperatures (0°C to 90°C). Thereafter, antifreeze activity was measured. As a result, 100 to 110% of antifreeze activity was found be to retained relative to activity of the sample held at 0°C, and stability with respect to heat was shown (FIG. 8).

[Working Example 5]

<Measurement of Recrystallization Inhibition Activity >

Ice crystals were observed by microscope, and the count of fine ice crystals was observed to determine recrystallization inhibition activity for ice crystals. That is to say, 2 µL of the present protein sample 1 dissolved in a 30% sucrose solution was placed drop-wise on a cover glass, and another cover glass was placed thereon to sandwich the solution therebetween. The cover glass assembly was placed on the stage of an optical microscope (model BH2 microscope, produced by Olympus Corp.) and a temperature controller (LK600 temperature controller, produced by Linkam Scientific Instruments Ltd.) was used to perform heating and cooling repeatedly at a rate of 0.1°C/second, in order, to temperatures of 20°C, -30°C, 20°C, -30°C, 20°C, -30°C, and -10°C. Then temperature was held at -10°C for 30 minutes, and the count was determined of fine ice crystals having a surface area of 10.01 to 35 $\mu m^2$. As a result, recrystallization inhibition activity for ice crystals was found to display a peak at a concentration of $1.0 \times 10^{-2}$ mg/mL as shown in FIG. 9.

[Working Example 6]

<Purification of the Present Protein Sample 1 from Various Parts of Antarctic Krill>

In order to check what part of the krill contains a large amount of the above mentioned present protein sample 1, eyeballs, meat, liver/pancreas, and shell were sorted, and the presence of protein was checked for each of the parts using SDS-polyacrylamide gel electrophoresis. Since a particularly large amount was found to be contained in the shell, 20 mg of protein was obtained from 10 g of shell of Antarctic krill using the same purification method as that of Working Example 1.

[Working Example 7]

<Measurement of Antifreeze Activity of Protein Derived from Crustaceans>

Antifreeze activity of crustacean-derived protein was observed for non-krill crustaceans. Using the same method as that of Working Example 1, protein was purified from the shells of king crab, snow crab, and American crayfish. As a result of performance of observations, antifreeze activity was confirmed (Table 1).

[Table 1]

|  | Raw material weight (g) | Extraction amount (mg) | Yield (%) | Antifreeze activity (°C)[*1] | Ice nucleation activity (°C)[*2] |
|---|---|---|---|---|---|
| Antarctic krill (shell) | 10 | 20 | 0.2 | 0.4 | -6.83 |
| snow crab (shell) | 20 | 2 | 0.01 | 0.48 | -9.2 |
| king crab (shell) | 30 | 3 | 0.01 | 0.24 | -6.83 |
| American crawfish (shell) | 12 | 6 | 0.05 | 0.3 | -5.6 |
| *1: Protein concentration = 1 mg/mL. *2: Protein concentration = 0.1 mg/mL. | | | | | |

[Working Example 8]

<Investigation of Method of Extraction of Antifreeze Protein from Antarctic Krill>

1. Extraction from Whole Krill

After pulverization of 2 kg of frozen krill using a food processor, 2 L of tap water was added to produce a suspension liquid. Thereafter, the produced suspension liquid was heated for 30 minutes at 80°C, and 2.6 L of a supernatant was recovered by centrifugal separation for 30 minutes at 5,000 G. This supernatant was concentrated under vacuum at 45°C to obtain 0.52 L of concentrate solution. This concentrate solution was dried using a spray dryer to obtain a powder (extract powder A). Weight of the obtained powder was 121 g, and the proportion of protein contained in this powder was 24.2%.

2. Extraction from Dried Krill Shells

After drying shells separated from krill, the shells were pulverized to obtain dried krill shell material. Then 7 L of tap water was added per 1 kg of the obtained dried krill shell material to produce a suspension liquid. Thereafter, the produced suspension solution was heated for 30 minutes at 80°C, and centrifugal separation at 5,000 G for 30 minutes was used to recover 4.7 L of supernatant. This supernatant was concentrated under vacuum at 45°C to obtain 0.86 L of concentrate solution. This concentrate solution was dried using a spray dryer to obtain a powder (extract powder B). Weight of the obtained powder was 124 g, and the proportion of protein contained in this powder was 10.6%.

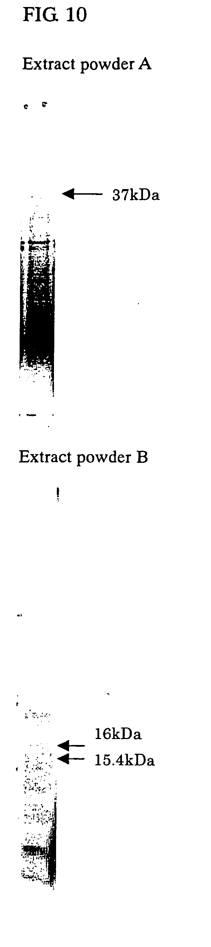

The above mentioned extract powders A and B were analyzed by SDS-polyacrylamide gel electrophoresis. The protein of 37 kDa molecular weight was found to be contained in extract powder A, and the proteins at 16 kDa and 15.4 kDa were found to be contained in extract powder B (FIG. 10).

<Measurement 1 of Antifreeze Activity of Extract Powders A and B>

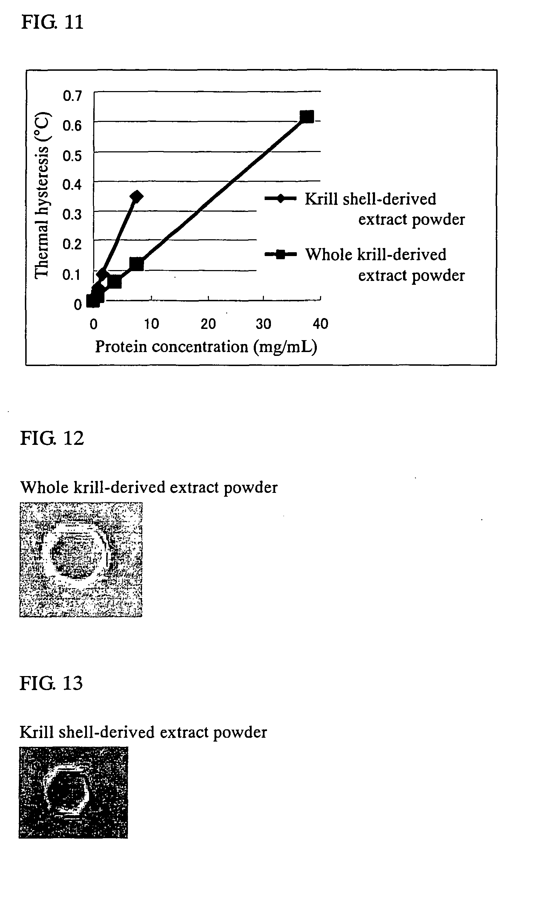

Antifreeze activity was observed for extract powders A and B. After suspension of the extract powder using distilled water, desalting was performed using ethanol precipitation treatment, and then thermal hysteresis was measured using an osmometer. Both extract powders were confirmed to increase thermal hysteresis in a manner dependent on the concentration of the protein (FIG. 11). Based on these results, a protein having antifreeze activity was shown to be contained in the krill-derived extract powders produced by the above mentioned extraction method. Moreover, when thermal hysteresis at 1 mg/mL was compared, as shown in Table 2, antifreeze activity of the extract powder B extracted from dried krill shell material was observed to be 3 times as strong (antifreeze activity) as the activity of the extract powder A.

[Table 2]

|  | Thermal hysteresis (°C/mg) |
| --- | --- |
| extract powder A derived from whole krill | 0.0163 |
| extract powder B derived from krill shell | 0.0472 |

<Measurement 2 of Antifreeze Activity of Extract Powders A and B>

The effect of suppression of growth of ice crystals by krill-derived extract powder was observed using a microscope. The extract powder suspended in distilled water was applied and sandwiched between 2 cover glasses in a cooled stage placed in a microscope. Temperature within the cooled stage was lowered down to -30°C and once the suspension liquid had frozen, temperature was raised to the vicinity of 0°C to produce ice crystal grains. Then temperature within the cooled stage was lowered at a rate of 1°C/minute, and the appearance of ice crystals growing in accompaniment with the temperature drop was observed. When the suspensions of the whole krill-derived extract powder A and the krill shell-derived extract powder B were used, as shown both in FIG. 12 and FIG. 13, growth of ice crystals was suppressed, and ice crystals were observed to have a hexagonal planar shape. Thus, both of these extract powders were shown to have an ice crystal growth suppression effect.

<Measurement of Recrystallization Inhibition Activity for Ice Crystals of the Extract Powders A and B>

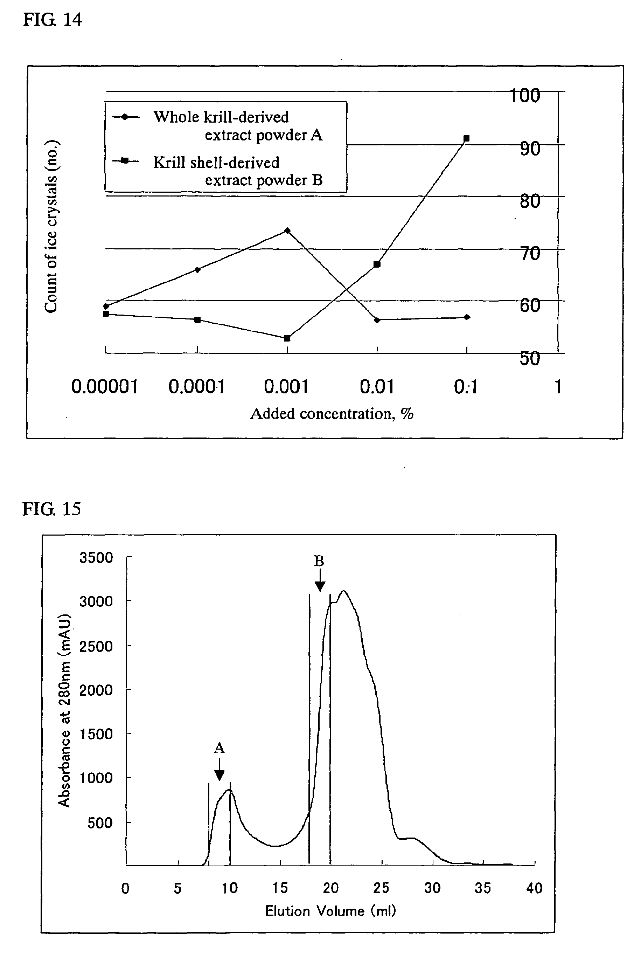

Ice crystal recrystallization inhibition effect due to the krill-derived extract powders was observed. That is to say, 2 $\mu$L of the present protein sample dissolved in 30% sucrose solution was placed drop-wise on a cover glass, and another cover glass was placed thereon to sandwich the solution therebetween. The cover glass assembly was placed on the stage of an optical microscope (model BH2 microscope, produced by Olympus Corp.) and a temperature controller (LK600 temperature controller, produced by Linkam Scientific Instruments Ltd.) was used for heating and cooling repeatedly at a rate of 0.1°C/second, in order, to temperatures of 20°C, -30°C, 20°C, -30°C, 20°C, -30°C, and -10°C. Then temperature was held at -10°C for 30 minutes, and the count was determined of fine ice crystals having a surface area of 10.01 to 35 $\mu$m$^2$. As shown in FIG. 14, the whole krill-derived extract powder A was found to have recrystallization inhibition activity that peaked at 0.001% concentration, and the krill shell-derived extract powder B was found to have recrystallization inhibition activity that depended on concentration.

<Extraction and Purification of Protein Having Antifreeze Activity from Antarctic Krill Dried Powder>

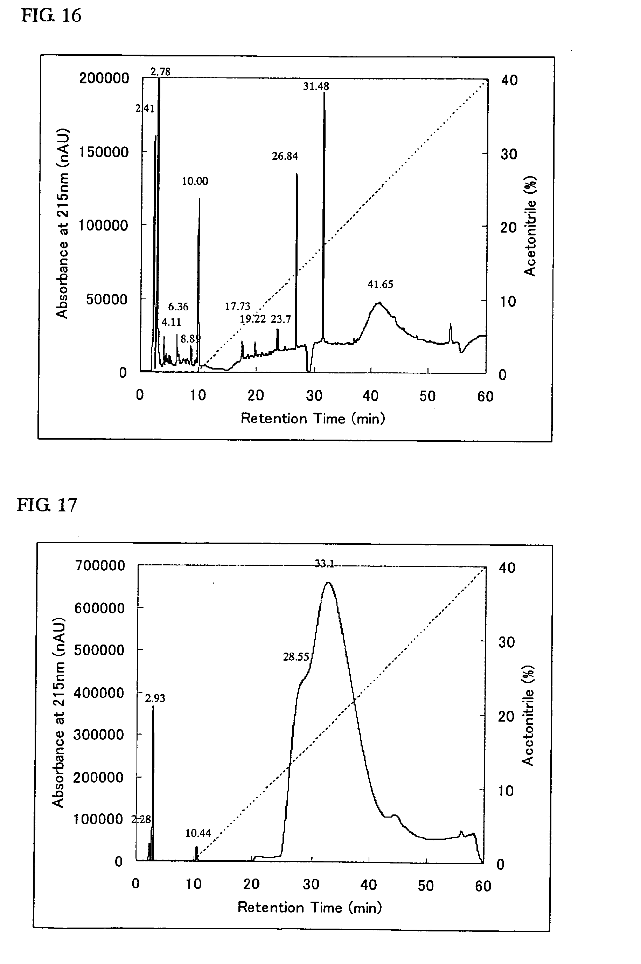

50 mL of an extract solution containing 20 mM Tris-HCl (pH 7.5) and 1 mM PMSF was added to 5 g of Antarctic krill dried powder. After suspension, extraction was performed for 30 minutes at 90°C. Thereafter, this suspension liquid was subjected to 30 minutes of centrifugal separation at 10,000 G to obtain a supernatant. Then this supernatant was applied for gel filtration chromatography using SUPERDEX G30 column (GE Healthcare) equilibrated with 20 mM Tris-HCl (pH 7.5). At this time, the presence of proteins having antifreeze activity in the chromatogram (shown in FIG. 15) in the 8 - 10 mL eluate (FIG. 15A) and 18 - 20 mL (FIG. 15B) eluate fractions was confirmed by change of morphology of ice crystals observed using a microscope and temperature controller. Then, these fractions were applied for reversed phase HPLC. That is to say, 20 μL of the respective fraction was adjusted using trifluoroacetic acid (0.1% final concentration) and was applied for HPLC (JASCO PU-1580, UV-1570, DG-1580-53, HG-1580-32, CO-1565) using an ODS column (MIGHTYSIL RP-18 GP, 150 - 4.6 mm) equilibrated with 0.1% trifluoroacetic acid. Proteins were separated and eluted by a linear gradient of 0 - 40% acetonitrile. These results are shown in FIG. 16 (8 - 10 mL eluate fraction) and FIG. 17 (18 - 20 mL eluate fraction). As shown in the figures, the present proteins had peaks that eluted at retention times of 2.41, 2.78, 4.11, 6.36, 8.89, 10.00, 17.73, 19.92, 23.70, 26.84, 31.48, and 41.65 minutes, or 2.28, 2.93, 10.44, 28.58, and 33.1 minutes, respectively.

Measurement conditions are listed below for the above mentioned reversed phase HPLC.

Column = MIGHTYSIL RP-18 GP, 150 - 4.6 mm

Eluting solvent:

0 - 10 minutes = 0.1% trifluoroacetic acid aqueous solution
10 - 60 minutes = 0.08% trifluoroacetic acid / 80% acetonitrile
Flow rate = 0.8 mL/minute
Column temperature = 25°C

[Working Example 9]

<Starch Gel Syneresis Prevention Effect Due to Addition of Krill-Derived Extract Powder>

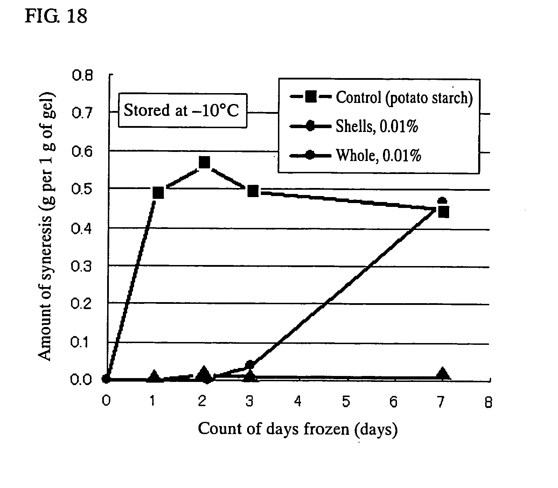

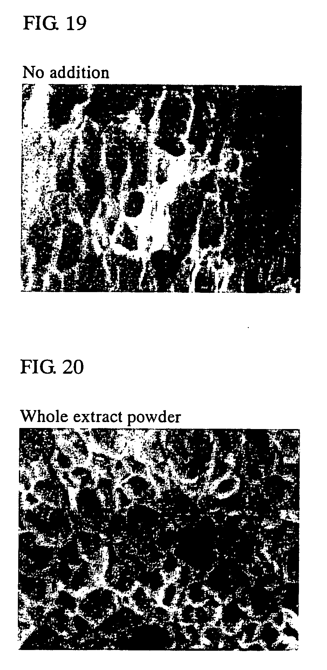

The starch gel syneresis prevention effect due to addition of krill-derived extract powder was observed. Potato starch was used as a raw material with a RVA (Rapid Visco Analyzer) to produce a starch gel. The starch gel was frozen at -20°C, stored at -10°C, and the appearance of syneresis was observed after thawing. As a result, addition of the krill-derived extract powder was confirmed to suppress an increase of the amount of syneresis (FIG. 18). Moreover, the starch gel after 7 days of frozen storage was freeze dried, and structure was observed using an electron microscope. As a result, the starch gel containing added extract powder was confirmed to have a fine structure in comparison to a gel without extract powder addition (FIGS. 19 and 20). This is thought to occur since the krill-derived extract powder suppresses growth of ice crystals during frozen storage of the starch gel and since the krill-derived extract powder suppresses deterioration of the starch gel during frozen storage.

[Working Example 10]

<Application to Seafood: Addition to Minced Meat of Alaska Pollack>

Meat was collected from Alaska pollock, which is a fresh fish caught off of the Sanriku Coast, and a 3 mm mesh mincer was used to form minced meat. Then 500 g of this minced meat was sampled, and 50 g of water and 50 g of sugar were added. The extract powder B was further added to give concentrations ranging from 0 to 0.1%. The mixture was stirred for 25 seconds using a tabletop mixer. This mixture was then used as the minced meat sample. Then, the respective samples were frozen overnight at -25°C and were stored thereafter for 2 weeks at -10°C. These samples were used for measurement of salt-induced solubility and degree of TMAO decomposition as indicators for evaluation of preservation ability.

Measurement of Salt-Induced Solubility

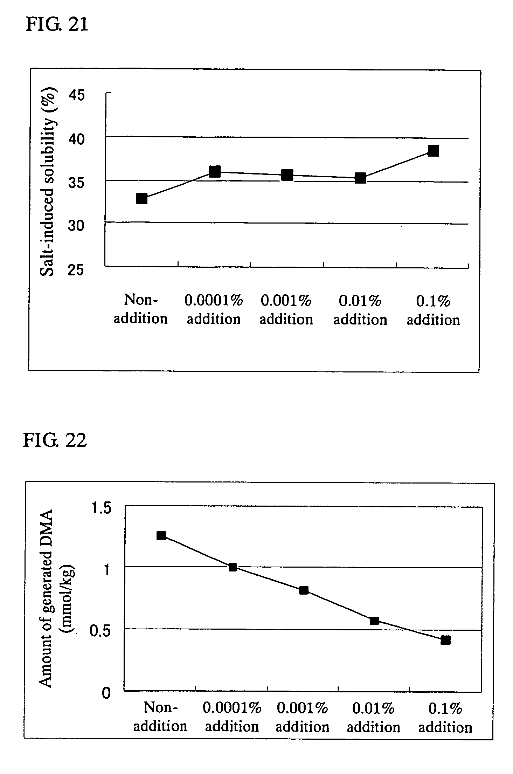

After addition of 27 mL of 0.1 M KCl 20 mM Tris-HCl (pH 7.5) solution to 3 g of the minced meat, the mixture was homogenized using a homogenizer (10,000 rpm × 30 seconds, 3 times). Thereafter, the mixture was subjected to centrifugal separation (5,000 rpm × 10 minutes) to obtain a precipitate. Then, an additional 27 mL of the 0.5 M KCl 20 mM Tris-HCl (pH 7.5) solution was added to this precipitate, and the mixture was stirred. Centrifugal separation was performed again (5,000 rpm × 10 minutes), and 27 mL of 0.5 M KCl 20 mM Tris-HCl (pH 7.5) solution was added to the obtained precipitate. The mixture was stirred, 30 μL of 1 M ATP was added, and the mixture was stored overnight on ice. The mixture thereafter was stirred, volume of the suspension solution was measured, and protein concentration of the suspension solution was measured by the biuret method. The suspension solution was subjected to further centrifugal separation (7,000 rpm × 15 minutes), and protein concentration of the obtained supernatant was measured by the biuret method. Volume was also measured. Based on the above measurements, salt-induced solubility was calculated from the ratio of the protein concentration in the previous supernatant over the protein concentration in the suspension liquid. As a result, as shown in FIG. 21, salt-induced solubility increased with increases in the added amount of the extract powder B, and due to addition of the extract powder B, a trend was shown for suppression of quality deterioration of minced meat of Alaska pollock during frozen storage.

Measurement of Decomposition Reaction of TMAO

After 10 mL of distilled water and 10 mL of 10% trichloroacetic acid (TCA) were added to 10 g of the minced meat sample, the mixture was homogenized (12,000 rpm × 2 minutes). Thereafter, centrifugal separation (7,500 rpm × 20 minutes) was used to obtain a supernatant. Then, 5 mL of 5% TCA solution was added to the precipitate, the mixture was well stirred, and centrifugal separation was used to obtain a supernatant. This operation was carried out 2 times. The heretofore obtained supernatants were placed in a 50 mL measuring flask, and 5% TCA solution was used to increase volume to the meniscus mark. This solution was used as the TCA extract liquid.
Then 1 mL of the TCA extract liquid and 3 mL of distilled water were mixed, 0.4 mL of copper-ammonia reagent and 5% carbon disulfide - benzene solution were added, and the mixture was incubated for 5 minutes at 40°C. Thereafter, the mixture was repeatedly shaken, and centrifugal separation (3,500 rpm × 5 minutes) was performed. Then, the obtained supernatant was transferred to a test tube containing about 0.2 g of anhydrous sodium sulfate, and optical absorbance of this solution was measured at 440 nm. The generated concentration of DMA is indicated by the following formula.

```
DMA amount (mM) = (value measured at 440 nm) ÷ 1.3 × 2 × 50 ÷
1000 ÷ (sample weight) × 1000
```

As shown in FIG. 22, the generated concentration of DMA due to decomposition of TMAO was shown to decline according to the added concentration of the extract powder B. Due to addition of the extract powder B, formaldehyde is generated at the same amount as DMS due to decomposition of TMAO. Suppression of the generated amount of DMA means suppression formaldehyde generation, and this indicates that freezing denaturation of Alaska pollock minced meat by formaldehyde was suppressed during frozen storage.

[Working Example 11]

<Application to Food>

1. Japanese Wheat Noodles

Japanese wheat noodles were made by mixing together and kneading the blend indicated in Table 3 (according to this blend, enriched product contained the present protein sample 2 in the Japanese wheat noodles at 0.00036% concentration). After rolling, the noodle strips were wrapped in vinyl and were aged for 1 h at room temperature. Thereafter, the noodle strips were subjected to further rolling, and were cut to form noodles. The noodles were boiled for 10 minutes in boiling water. Thereafter, the noodles were cooled for 5 minutes in flowing water. The noodles were transferred to a colander and were dewatered for 5 minutes. Thereafter, the noodles were frozen overnight at -20°C. Thereafter, the noodles were transferred to -10°C storage and were stored frozen for 2 weeks. The noodles were thawed naturally for about 2 h at room temperature, and food texture of the noodles was evaluated. As a control, Japanese wheat noodles were also prepared that did not contain the protein of the present invention and a comparison was performed. As a result, in comparison to the control, as shown in Table 3, the Japanese wheat noodles containing the present protein sample 2 had a smooth noodle surface, and food texture was springy and elastic.
[Table 3]

| Frozen Japanese wheat noodle blend | Comparative product | Enriched product |
|---|---|---|
| wheat flour for Japanese wheat noodles | 500 g | 500 g |
| table salt | 12.5 g | 12.5 g |
| water | 210 g | 137.7 g |
| 10% solution of the freeze dried material of Working Example 2 | - | 72.3 g |

(continued)

| Frozen Japanese wheat noodle blend | Comparative product | Enriched product |
|---|---|---|
| smoothness of the noodle surface | bad | good |
| springy feel | bad | good |
| elasticity | average | good |

2. Cooked Rice

Cooked rice was prepared from the blend indicated in Table 4 (according to this blend, the enriched product contained the present protein sample 2 in the cooked rice at a concentration of 0.00036%). To polished white rice (Kirara 397, rice produced in 2005), water (1.35-fold amount) was added, and the rice was cooked using a domestic rice cooker. Then after cooking, the rice was left to rest for 30 minutes and was molded into 10 pieces (20 g each) of sushi. Thereafter, the cooked rice was frozen for 3 days at -20°C, was then transferred to -10°C storage, and was stored frozen for 5 days. The cooked rice was thawed naturally for about 2 h at room temperature, and food texture of the cooked rice was evaluated. As a result, in comparison to the control, as shown in Table 4, the enriched product had adhesion and good food texture.

[Table 4]

| Cooked rice blend | Comparative product | Enriched product |
|---|---|---|
| rice (Kirara, produced in 2005) | 300 g | 300 g |
| water | 405 g | 334.5 g |
| 10% solution of the freeze dried material of Working Example 2 | - | 70.5 g |
| adhesion | average | good |
| surface gloss | good | good |

3. Steamed Egg Custard-Stew (Chawan-mushi)

Steamed egg custard-stew was prepared from the blend indicated in Table 5 (according to this blend, the enriched product contained the present protein sample 2 in the steamed egg custard-stew at a concentration of 0.00033%). After the materials were blended, 60 g portions were placed in heat resistance containers, were placed in a steamer, and were steamed. After crude heat removal at room temperature, the steamed egg custard-stew was frozen for 3 days at -20°C and was then stored frozen for 5 days at -10°C. For evaluation, the steamed egg custard-stew was steamed for 10 minutes using a steamer, and food texture was evaluated.
As a result, in comparison to the control, the enriched product had smooth food texture without incorporation of voids, the enriched product was free of dripping, and flavor of the enriched product was good.

[Table 5]

| Steamed egg custard-stew blend | Comparative product | Enriched product |
|---|---|---|
| egg | 180 g | 180 g |
| instant bouillon (granules) | 3g | 3g |
| table salt | 3g | 3g |
| soy sauce | 3g | 3g |
| water | 467 g | 407 g |
| 10% solution of the freeze dried material of Working Example 2 | - | 60 g |
| smoothness | average | good |
| condition of voids | bad | good |

[INDUSTRIAL APPLICABILITY]

The protein of the present invention is contained in large amounts in shells of crustaceans which are mostly discarded except for use as meal, chitin, and chitosan raw material. Thus, large volume preparation and inexpensive production is possible. Moreover, application by addition to foods is easy since this is a food-derived protein. Since the protein of the present invention has antifreeze activity and recrystallization inhibition activity for ice crystals, wide use is possible with the object of quality maintenance and the like of frozen food and the like.

[Sequence Listing Free Text]

SEQ ID No. 1
N-terminal amino acid sequence
SEQ ID No. 2
N-terminal amino acid sequence
SEQ ID No. 3
N-terminal amino acid sequence
SEQ ID No. 4
N-terminal amino acid sequence

[Sequence Listing]

<110> Nippon Suisan Kaisha, Ltd.

<120> Crustacean-derived protein having antifreeze activity

<130> YCT-1258

<141> 2007-03-13

<150> JP 2006-67758

<151> 2006-03-13

<160> 4

<210> 1

<211> 20

<212> PRT

<213> Antarctic krill (Euphausiacea sp.)

<400> KYGGEFPARPDNIXFENVDG (one letter code)

           Lys Tyr Gly Gly Glu Phe Pro Ala Arg Pro Asp Asn

Ile Xaa Phe Glu Asn Val Asp Gly (three letter code)

<210> 2

<211> 19

<212> PRT

<213> Antarctic krill (Euphausiacea sp.)

<400> TTYKYGGEFPARPDNXXFE (one letter code)

Thr Thr Tyr Lys Tyr Gly Gly Glu Phe Pro Ala Arg
Pro Asp Asn Xaa Xaa Phe Glu (three letter code)


<210> 3

<211> 20

<212> PRT

<213> Antarctic krill (Euphausiacea sp.)

<400> DYDVAHEQQDVNAFLTKITG (one letter code)

Asp Tyr Asp Val Ala His Glu Gln Gln Asp Val Asn Ala Phe
Leu Thr Lys Ile Thr Gly (three letter code)


<210> 4

<211> 18

<212> PRT

<213> Antarctic krill (Euphausiacea sp.)

<400> SDPKFQQDINTRLXNVYE (one letter code)

Ser Asp Pro Lys Phe Gln Gln Asp Ile Asn Thr Arg Leu
Xaa Asn Val Tyr Glu (three letter code)


**Claims**

1. A crustacean-derived protein having antifreeze activity.

2. The protein having antifreeze activity according to claim 1; wherein
   the crustacean-derived protein, as measured by molecular weight measurement using sodium dodecyl sulfate (SDS) - polyacrylamide gel electrophoresis, has reduced-form molecular weights of about 37,000, about 16,000, and/or about 15,400; and
   the N-terminal amino acids are indicated by SEQ ID No. 1 or SEQ ID No. 2.

3.  The protein having antifreeze activity according to claim 1 or claim 2; wherein the crustacean is a shrimp, crayfish, crab, krill, or mantis shrimp.

4.  A crustacean extract having antifreeze activity and containing a crustacean-derived protein having antifreeze activity.

5.  The crustacean extract having antifreeze activity of claim 4; wherein
    the crustacean-derived protein, as measured by molecular weight measurement using sodium dodecyl sulfate (SDS) - polyacrylamide gel electrophoresis, has reduced-form molecular weights of about 37,000, about 16,000, and/or about 15,400; and
    the N-terminal amino acids are indicated by SEQ ID No. 1 or SEQ ID No. 2.

6.  The crustacean extract having antifreeze activity of claim 4 or claim 5; wherein the crustacean is a shrimp, crayfish, crab, krill, or mantis shrimp.

7.  A production method for a crustacean extract having antifreeze activity **characterized by** performing hot water extraction from the shells, meat, and/or internal organs of the crustacean.

8.  The production method for a crustacean extract according to claim 7; wherein the hot water extraction is performed using hot water of a temperature greater than or equal to 60°C.

9.  A production method for a crustacean extract having antifreeze activity; wherein the production method comprises: performing heat treatment and/or reduction treatment of a body liquid obtained by compressing the meat and/or internal organs of the crustacean or of an extract liquid of the shells, meat, and/or internal organs.

10. The production method for a crustacean extract having antifreeze activity according to claim 9; wherein the body liquid obtained by compression of the meat and/or internal organs of the crustacean, or the extract liquid of the shells, meat, and/or internal organs, as measured by molecular weight measurement using sodium dodecyl sulfate (SDS) - polyacrylamide gel electrophoresis, has a non-reduced-form molecular weight of about 200,000 and displays reduced-form bands at about 86,000 and 90,000; and
    the N-terminal amino acids are indicated by SEQ ID No. 3 or SEQ ID No. 4.

11. The production method for a crustacean extract having antifreeze activity according to claim 9 or claim 10; wherein extraction from the shells, meat, and/or internal organs of the crustacean is performed by extraction using an extraction liquid having an added surfactant.

12. The production method for a crustacean extract having antifreeze activity according to claim 9, claim 10, or claim 11; wherein the heat treatment is performed at a temperature greater than or equal to 60°C; and the reduction treatment is performed by addition of a reducing agent.

13. The production method for a crustacean extract having antifreeze activity according to any one of claims 7 through 12; wherein the crustacean is a shrimp, crayfish, crab, krill, or mantis shrimp.

14. A quality improvement agent for food that includes:

    the protein having antifreeze activity of any one of claims 1 through 3;
    the crustacean extract having antifreeze activity of any one of claims 4 through 6; or
    the crustacean extract having antifreeze activity produced by the method of any one of claims 7 through 13.

15. A food of improved quality to which has been added the quality improvement agent of claim 14.

16. The food according to claim 15; wherein the food is a frozen food.

17. The food according to claim 15 or claim 16; wherein the food uses egg, wheat flour, or fish meat as a raw material.

FIG. 1

FIG. 2

FIG. 3

FIG. 4

FIG. 5

37kDa

16kDa
15.4kDa

FIG. 6

FIG. 7

FIG. 8

FIG. 9

FIG. 10

Extract powder A

← 37kDa

Extract powder B

16kDa
15.4kDa

FIG. 11

FIG. 12

Whole krill-derived extract powder

FIG. 13

Krill shell-derived extract powder

FIG. 14

Legend:
- Whole krill-derived extract powder A
- Krill shell-derived extract powder B

Y-axis: Count of ice crystals (no.)
X-axis: Added concentration, %

FIG. 15

Y-axis: Absorbance at 280nm (mAU)
X-axis: Elution Volume (ml)

FIG. 16

FIG. 17

FIG. 18

FIG. 19

No addition

FIG. 20

Whole extract powder

FIG. 21

FIG. 22

| INTERNATIONAL SEARCH REPORT | International application No. |
|---|---|
| | PCT/JP2007/054989 |

**A.   CLASSIFICATION OF SUBJECT MATTER**
*C07K14/435*(2006.01)i, *A23J1/04*(2006.01)i, *A23J3/04*(2006.01)i, *A23L3/36* (2006.01)i, *A23B4/08*(2006.01)n, *A23L1/10*(2006.01)n, *A23L1/16*(2006.01)n, *A23L1/32*(2006.01)n, *A23L1/325*(2006.01)n
According to International Patent Classification (IPC) or to both national classification and IPC

**B.   FIELDS SEARCHED**

Minimum documentation searched (classification system followed by classification symbols)
C07K14/435, A23J1/04, A23J3/04, A23L3/36, A23B4/08, A23L1/10, A23L1/16, A23L1/32, A23L1/325

Documentation searched other than minimum documentation to the extent that such documents are included in the fields searched
Jitsuyo Shinan Koho          1922–1996   Jitsuyo Shinan Toroku Koho   1996–2007
Kokai Jitsuyo Shinan Koho    1971–2007   Toroku Jitsuyo Shinan Koho   1994–2007

Electronic data base consulted during the international search (name of data base and, where practicable, search terms used)
BIOSIS/MEDLINE/WPIDS(STN), GenBank/EMBL/DDBJ/GeneSeq, UniProt/Geneseq

**C.   DOCUMENTS CONSIDERED TO BE RELEVANT**

| Category* | Citation of document, with indication, where appropriate, of the relevant passages | Relevant to claim No. |
|---|---|---|
| A | WO 2004/072283 A1  (NAT. INST. ADVANCED IND. SCI. & TECHNOLOGY), 26 August, 2004 (26.08.04), & JP 2005-505033 A | 1-17 |
| A | WO 1999/037673 A2  (UNILEVER N.V. et al.), 29 July, 1999 (29.07.99), & JP 2002-508303 A      & EP 1049713 B1 & US 6774210 B1 | 1-17 |
| A | WO 1997/002343 A1  (UNILEVER N.V. et al.), 23 January, 1997 (23.01.97), & JP 11-508451 A      & JP 2004-043484 A & EP 0836646 B1      & EP 1614753 A2 & US 6914043 B1      & US 2005/0205833 A1 | 1-17 |

☒   Further documents are listed in the continuation of Box C.          ☐   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 May, 2007 (10.05.07) | 22 May, 2007 (22.05.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
|---|
| PCT/JP2007/054989 |

C (Continuation).    DOCUMENTS CONSIDERED TO BE RELEVANT

| Category* | Citation of document, with indication, where appropriate, of the relevant passages | Relevant to claim No. |
|---|---|---|
| A | WO 1999/000493 A1  (UNIV. QUEENS KINGSTON),<br>07 January, 1999 (07.01.99),<br>& JP 2002-507889 A       & EP 0990032 A1<br>& US 6392024 B1       & US 6747130 B2 | 1-17 |
| A | WO 2004/104201 A1  (NAT. INST. ADVANCED IND.<br>SCI. & TECHNOLOGY),<br>02 December, 2004 (02.12.04),<br>& JP 2004-344033 A | 1-17 |
| A | JP 2001-245659 A  (IKEDA SHOKKEN KABUSHIKI<br>KAISHA),<br>11 September, 2001 (11.09.01),<br>Particularly, Par. Nos. [0003] to [0005]<br>(Family: none) | 1-17 |
| A | JP 2004-000019 A  (IKEDA SHOKKEN KABUSHIKI<br>KAISHA),<br>08 January, 2004 (08.01.04),<br>(Family: none) | 1-17 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9804148 A
- WO 9937673 A
- WO 9611586 A
- WO 9702343 A
- WO 9900493 A
- WO 9013571 A

**Non-patent literature cited in the description**

- *Appl. Microbiol.,* 1974, vol. 28, 456
- *Proc. 4th Int. Cont. Plant. Path. Bact.,* 1978, 725